# EUROPÄISCHE PATENTSCHRIFT

(11) **EP 0 579 622 B1**
(45) Veröffentlichungstag und Bekanntmachung des Hinweises auf die Patenterteilung: **24.01.1996**
(21) Anmeldenummer: 92906622.3
(22) Anmeldetag: 19.03.1992
(51) Int. Cl.: C07C 43/23, A61K 31/09, A61K 31/12, C07C 49/255

(54) **NEUE PHENOLETHER, VERFAHREN ZU IHRER HERSTELLUNG UND ARZNEIMITTEL, DIE DIESE VERBINDUNGEN ENTHALTEN**
NEW PHENOL ETHERS, PROCESS FOR PRODUCING THE SAME AND MEDICAMENTS CONTAINING THESE COMPOUNDS
NOUVEAUX ETHERS PHENOLIQUES, LEUR PROCEDE DE PREPARATION ET MEDICAMENTS CONTENANT CES COMPOSES

(30) Priorität: 23.03.1991 DE 4109627
(43) Veröffentlichungstag der Anmeldung: 26.01.1994
(73) Patentinhaber: BOEHRINGER MANNHEIM GMBH, D-68298 Mannheim (DE)
(72) Erfinder: WITTE, Ernst-Christian, D-6800 Mannheim 1 (DE); WOLFF, Hans, Peter, D-6800 Mannheim 1 (DE); DRESEL, Alois, D-6905 Schriesheim (DE)
(74) Vertreter: Weber, Manfred, Dr.
(86) Internationale Anmeldenummer: EP9200602
(87) Internationale Veröffentlichungsnummer: WO9216485

(56) Entgegenhaltungen:
- WO-A-91/01124
- US-A- 4 115 590

## Beschreibung

Gegenstand der vorliegenden Anmeldung sind Phenolether der allgemeinen Formel I 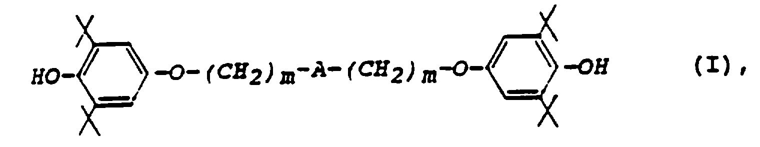 in welcher
- m =: eine ganze Zahl zwischen 1 und 6 und
- A =: eine Bindung, eine Methylengruppe, eine Ketogruppe oder eine Gruppe -CH(OH)-
darstellen, mit Ausnahme der Verbindung 1,2-Bis-[4-hydroxy-3,5-di-tert.butyl-phenoxy]ethan, Verfahren zu Ihrer Herstellung sowie Arzneimittel, die diese Verbindungen enthalten.

Die Verbindungen der Formel I sind bis auf drei Ausnahmen noch nicht beschrieben: In der CH-A-56624 ist die Verbindung I mit m = 3, A = Bindung als Stabilisator für Polyamide und in C.A. 75, 64588d die Verbindung I mit m = 2, A = Bindung als Inhibitor der Polymerisation von Vinylmonomeren beschrieben. In dem US-A-4,115,590, welches zweikernige Phenole betrifft, die eine Verminderung des Plasmalipidspiegels bewirken, ist eine allgemeine Formel offenbart, welche die Verbindung 1,2-Bis[4-hydroxy-3,5-di-tert.butyl-phenoxy]ethan umfaßt. Die genannte Verbindung, ihre Herstellung und Verwendung wird deshalb nicht beansprucht. Die beiden erstgenannten Verbindungen werden aus dem Stoffanspruch der vorliegenden Anmeldung ausgeschlossen. Nicht ausgeschlossen ist dagegen ihre Verwendung als pharmakologisch wirksame Substanzen im Sinne der folgenden Erläuterungen, da eine derartige Verwendung in den beiden genannten Patentschriften nicht beschrieben wird.

Die Verbindungen der allgemeinen Formel I erfüllen alle Voraussetzungen für ein wirksames Arzneimittel gegen die vorzeitige Atheromatose und die progressive Arteriosklerose.

Sie verfügen aufgrund ihrer 2,6-Di-tert.butyl-phenylgruppe über eine starke Antioxidantienaktivität. Die Lipophilie dieser Antioxidantiengruppe bedingt, daß die Verbindungen sich im atherogenen low-density lipoprotein (LDL) anreichern und die sensitiven Bestandteile des LDL wirksam gegen reaktive Sauerstoffspezies schützen. Damit wird aber der LDL-Influx in die makrophagozytären Schaumzellen deutlich abgebremst, denn die pathologisch gesteigerte Aufnahme von atherogenem LDL in den Atheromzellen setzt dessen oxidative Modifikation voraus.

Sie sind Senker der Plasmalipide, indem sie wie Probucol die Plasmaclearance des LDL beschleunigen.

Die Substanzen werden weit besser als Probucol absorbiert. Sie verhindern dadurch die schaumzellige Degeneration der Macrophagen der Arterienwand bei einer deutlich geringeren Dosis.

Die Verbindungen haben therapeutischen Nutzen auch bei weiteren Erkrankungen mit pathologischer Lipidoxidation. Sie verhindern Reperfusionsschäden in ischämischen Geweben und können antiarrhytmisch wirken, da am Herzen eine starke arrhythmische Aktivität vor allem unter den Bedingungen der Reperfusionsischämie entsteht. Außerdem stabilisieren sie Gewebe-Lipoproteine, z. B. den Lungensurfectant, und sie wirken antiinflammatorisch und cytoprotektiv.

Die Herstellung der neuen Verbindungen der allgemeinen Formel I erfolgt in der Weise, daß man
a) Zwei Mol 3,5-Di-tert.butyl-hydrochinon (II) mit einem Mol einer Verbindung III zur Umsetzung bringt: 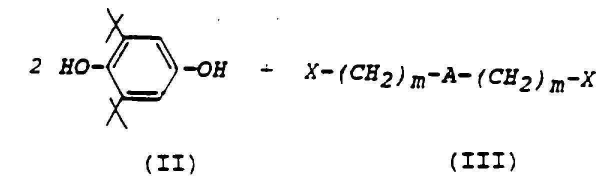 In Formel (III) bedeutet X eine reaktive Gruppe, und A und m haben die oben angegebene Bedeutung oder
b) zunächst ein Mol 3,5-Di-tert.butyl-hydrochinon (II) mit einem Mol einer Verbindung IV zur Umsetzung bringt 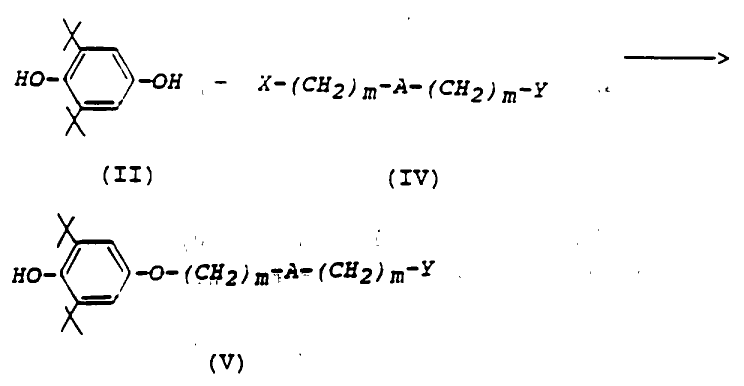 und anschließend die erhaltene Verbindung V gegebenenfalls nach Umwandlung der Gruppe Y in eine reaktive Gruppe X mit einem weiteren Mol II zur Reaktion bringt. In den Formeln IV und V haben X, m und A die oben angegebene Bedeutung, und Y bedeutet eine Gruppe, die selbst nicht zur Kondensation mit einem Phenol befähigt ist, die aber leicht in eine aktive Gruppe X umzuwandeln ist oder
b 2) für den speziellen Fall, daß m = 1 und A = CHOH bedeuten, kommt eine Umsetzung der Verbindung II mit Epichlorhydrin oder Epibromhydrin zur Verbindung VI infrage: 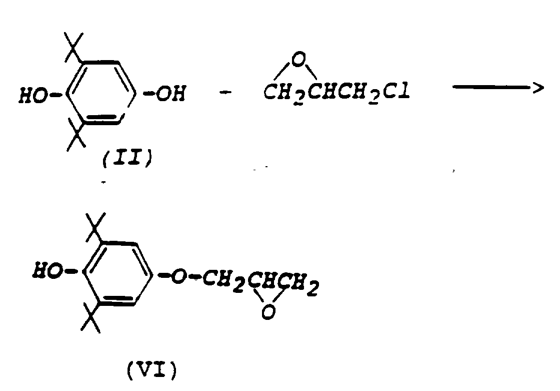 Die erhaltene Verbindung (VI) wird nun mit einem weiteren Mol II zum Di-ether umgesetzt.

Für die nachträgliche Umwandlung einer Gruppe A in eine andere Gruppe A bieten sich folgende Verfahren an:
1)Die Umwandlung einer Gruppe A - CO in eine Gruppe CH₂ ist durch Reduktion möglich.
2)Auch die Umwandlung von A = -CHOH- zu A = -CH₂- erfolgt durch Reduktion, gegebenenfalls nach Umwandlung der Gruppe A = -CHOH- in eine Gruppe -CHHal-, wobei Hal die Bedeutung Chlor oder Bröm hat.
3) Umgekehrt ist es möglich, eine Gruppe A = -CHOH- Oxidativ in eine Gruppe A = CO umzuwandeln.

Bevorzugt im Sinne des Erfindungsgedankens sind solche Verbindungen I, in denen m die Bedeutung 1 bis 3 hat und A eine Methylengruppe darstellt, wobei besonders bevorzugt ist eine Verbindung I, in der m = 1 und A = CH₂.

Bevorzugt sind weiterhin solche Verbindungen I, in denen A eine Ketogruppe oder eine Gruppe -CHOH- darstellt und m eine ganze Zahl zwischen 1 und 3 darstellt.

Als reaktive Gruppen der allgemeinen Formeln III und IV sind die Halogene Chlor und Brom sowie Sulfonsäureester-Gruppen wie z.B. der Mesyloxy- oder der Tosyloxy-Rest zu nennen.

Als Gruppe Y der allgemeinen Formeln IV und V ist bevorzugt die Hydroxylgruppe, es kommen aber auch alle anderen Gruppen infrage, die sich gegebenenfalls in mehreren Schritten in eine aktive Gruppe X umwandeln lassen, also z.B. die Carbaldehyd-Gruppe, die Carboxy- und-die Alkoxycarbonylgruppe. Die Umsetzung zwischen Di-tert.butylhydrochinon und einer Verbindung der allgemeinen Formel III erfolgt vorzugsweise in Gegenwart starker Basen wie Natronlauge oder Kalilauge, entweder ohne Lösungrmittel oder in Gegenwart von Ethanol oder Methanol.

Als Basen sind aber auch z.B. Natrium- oder Kaliumcarbonat oder organische Basen wie Tetraalkyl-ammoniumhydroxide einsetzbar.

Um unerwünschte Nebenreaktionen weitgehend auszuschließen, wird für das Verfahren a) eine Verfahrensweise bevorzugt, bei der zu dem Gemisch der beiden Reaktionspartner in Ethanol bei Siedehitze sehr langsam 5N-Natronlauge zugetropft wird.

Die Umsetzung der Verbindung II mit einer Verbindung IV zu einer Verbindung V gemäß b) wird in einer zu Verfahren a) analogen Weise vorgenommen.

Die Umwandlung der Gruppe Y in eine reaktive Gruppe X erfolgt nach an sich literaturbekannten Verfahren: Stellt Y eine Hydroxylgruppe dar, so ist eine Umwandlung in X = Br z.B. dadurch zu erreichen, daß man die Hydroxyverbindung mit Mesylchlorid in das Mesylat umwandelt und dieses mit Lithiumbromid in Aceton zur Bromverbindung umsetzt.

Für die Umsetzung der Verbindung II mit Epichlorhydrin gemäß b. 2) verwendet man zweckmäßig einen großen Überschuß an letzterem. Die Reaktion wird unter Schutzgas und in Gegenwart einer wasserfreien Base, bevorzugt pulv. Kaliumcarbonat, durchgeführt. Die so erhaltene Verbindung (VI) wird dagegen bevorzugt mit einer äquimolaren Menge II in Gegenwart von katalytischen Mengen einer starken Lauge wie z.B. Natronlauge zum Di-ether (I), m = 1, A - CHOH, umgesetzt.

Für die nachträgliche Umwandlung einer Gruppe A = CO in eine Gruppe A = CH₂ bevorzugt sind Reduktionsverfahren, die ohne Anwesenheit von starken Säuren auskommen, also z.B. die Umsetzung mit Hydrazinhydrat in Gegenwart von starkem Alkali (z.B. Kaliumhydroxid).

Für die nachträgliche Umwandlung einer Gruppe A = CO in eine sekundäre Alkoholgruppe ist bevorzugt die Reduktion mit komplexen Metallhydriden wie z.B. LiAlH₄ in Ether oder Tetrahydrofuran oder NaBH₄ in wäßrig-alkoholischem Medium.

Umgekehrt läßt sich eine Gruppe A = -CHOH- auch zur Ketogruppe oxidieren. Exemplarisch für eine derartige Oxidation seien die in Beispiel 3 genannten Bedingungen.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusäzte sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht.

Normalerweise sind 0.5 bis 40 und vorzugsweise 1.0 bis 20 mg/kg in einer oder mehreren Anwendungen pro Tag wirksam, um die gewünschten Resultate zu erhalten.

Bevorzugte Verbindungen im Sinne der vorliegenden Anmeldung sind:
2.1,5-Bis-[4-hydroxy-3,5-di-tert.butyl-phenoxy]-3-hydroxy-pentan
3.1,5-Bis-[4-hydroxy-3,5-di-tert.butyl-phenoxy]-3-oxo-pentan
4.1,7-Bis-[4-hydroxy-3,5-di-tert.butyl-phenoxy]-4-hydroxy-heptan
5.1,7-Bis-(4-hydroxy-3,5-di-tert.butyl-phenoxy]-4-oxo-heptan

Die nachfolgenden Beispiele zeigen einige der zahlreichen Verfahrensvarianten, die zur Synthese der erfindungsgemäßen Verbindungen verwendet werden können. Sie sollen jedoch keine Einschränkung im Sinne des Erfindungsgedankens darstellen.

### Beispiel 1

### 1,3-Bis-[4-hydroxy-3,5-di-tert.butyl-phenoxy]propan

Eine Lösung aus 39.0 g (175.4 mmol) 2,6-Di-tert.butyl-hydrochinon, 17.7 g (87.7 mmol) 1,3-Dibrompropan und 250 ml Ethanol wird unter Stickstoffatmosphäre zum Sieden erhitzt. Man tropft innerhalb drei Stdn. 38.6 ml 5N-NaOH zu, hält weitere drei Stdn. auf Siedetemperatur und kühlt dann ab. Dann wird mit 6N-HCl schwach angesäuert. Man dampft das Ethanol ab, gibt Wasser zu und extrahiert mit Ether. Die Etherphase wird getrocknet (Na₂SO₄) und eingedampft. Man digeriert im Ultraschallbad den Rückstand mit 90proz. Methanol, saugt ab, wäscht mit kaltem 90proz. Methanol nach und trocknet über KOH. Ausb. 17.0 g (40 % d.Th.); Schmp. 125°C.

In analoger Weise wird dargestellt:
1,10-Bis-(4-hydroxy-3,5-di-tert.butyl-phenoxy)decan
aus 2,6-Di-tert.butyl-hydrochinon und 1,l0-Dibromdecan. Ausb. 52 % d.Th.; Schmp. 100-l01°C (Ligroin).

### Beispiel 2

### 1,3-Bis-[4-hydroxy-3,5-di-tert.butyl-phenoxy]-2-hydroxy-propan

a) 1-(4-Hydroxy-3,5-di-tert.butylphenoxy)-2,3-epoxypropan
   Ein Gemisch aus 50.6 g (0.227 mol) 2,6-Di-tert.butyl-hydrochinon, 31.4 g (0.227 mol) pulv. Kaliumcarbonat sicc. und 680 ml Epichlorhydrin wird unter Stickstoff 48 Stdn. auf Rückflußtemperatur gehalten, dann dampft man weitgehend ein und nimmt den Rückstand in Ether auf. Die Etherphase wird mehrfach mit Wasser extrahiert, dann getrocknet und eingedampft. Der Rückstand (quant. Ausbeute), ein farbl. Oel, wird roh in die nächste Reaktion eingesetzt.
b) Titelverbindung
   Ein Gemisch aus 56.6 g (0.2 mol) des nach a) erhaltenen Epoxids, 44.4 g (0.2 mol) 2,6-Di-tert.butyl-hydrochinon und 16 Tropfen 10 N-NaOH wird 16 stdn. unter Stickstoff auf Rückflußtemperatur gehalten, wobei die Reaktionsmasse fest wird. Man löst in 200 ml heißem Methanol, filtriert u und fügt bis zur beginnenden Trübung Wasser zu. Nach dem Erkalten werden ausgefallene Kristalle abgesaugt, mit einem Gemisch aus 250 ml Methanol und 100 ml Wasser mehrmals gewaschen und dann getrocknet. Nach Umkristallisieren aus einem Gemisch aus Methanol und Wasser (4:1 Vol.) erhält man 68 g (68 % d.Th.) Produkt mit dem Schmp. 140-142°c.

### Beispiel 3

### 1,3-(4-Hydroxy-3,5-di-tert.butyl-phenoxy)-2-oxo-propan

Zu einem Gemisch aus 16.0 g (32 mmol) 1,3-(4-Hydroxy-3,5-di-tert.butyl-phenoxy)-2-hydroxypropan, 240 ml Ether, 19.8 g (96 mmol) Dicyclohexyl-carbodiimid, 9 ml Dimethylsulfoxid und 1.5 ml (18.6 zmol) Pyridin setzt man 1.2 ml (15.7 mmol) Trifluoressigsäure unter Eiskühlung zu, läßt über Nacht bei Raumtemperatur stehen und tropft dann innerhalb 30 min eine Lösung aus 9.6 g (106 mmol) Oxalsäure in 60 ml Methanol zu. Nach weiteren 30 min Rühren saugt man ab und löst die Kristalle in Ether. Die Etherlösung wird dreimal mit NaHCO₃-Lösung ausgeschüttelt, dann mit MgSO₄ getrocknet und eingedampft. Man kristallisiert aus Ligroin unter Zusatz von Aktivkohle um. Ausb.: 10.7 g (67 % d.Th.), Schmp. 124-126°C.

### Pharmakologischer Versuchsbericht

Die aufgeführten Verbindungen erfüllen alle Voraussetzungen für ein neues hochpotentes Antioxidans bzw. eines LDL assoziierten Lipoxidasehemmers gegen die vorzeitige Atheromatose und die progressive Arteriosklerose, da die durch ihre Anreicherung im LDL und starke Hemmwirkung gegenüber potentiell bei der LDL-Modifikation entscheidenden Lipoxydasen der Zielzellen des LDL im Atherom ihre Wirkung stärker als das bisher bekannte Probucol entfalten.

Die neuen Phenolether wirken LDL-protektiv. Bei Inkubation des LDL it Redoxmetallen hemmen sie die Bildung von thiobarbitursäure-reaktiven Lipidperoxiden (Tab. 1), bei der Oxidation des LDL durch Lipoxydase wird eine selektive Hemmwirkung deutlich (Tab. 2). Unter Testbedingungen, die zu einem wirksamen LDL-Schutz führen, wird die Cholesterinesterbildung in kultivierten Makrophagen gehemmt (Tab. 3).

Beim arteriosklerotischen Kaninchen ist die Wirkung der neuen Phenolether direkt am arteriosklerotischen Plaque nachzuweisen. 131I-TDS-LDL, ein Radioiod-LDL, das von den Zellen nach oxydativer Modifikation aufgenommen wird, akkumuliert nach i.v. Gabe massiv im Plaquegewebe. Die neuen Phenolether hemmen signifikant und massiv den LDL-Katabolismus und damit die Cholesterinüberladung der Arterienwandzellen. Daraus resultiert langfristig die Hemmung des Wachstums der Läsionen (Tab. 4).

In sämtlichen Versuchen wird mit Probucol-4,4'[Isopropylidendithio]-bis(2,6-di-tert.-butyl) verglichen.

**Tabelle 1**

| Inhibition der Cu++-abhängigen LDL-Oxidation | | |
|---|---|---|
| Testsubstanz | Zeit Std. | TBARS (OD 532) % |
| Kontrolle | 16 | 75 |
| | 23 | 100 |
| | | |
| Probucol | 16 | 56 |
| | 23 | 88 |
| | | |
| Verb. gem. Bsp.1 | 16 | 19 |
| | 23 | 76 |

125 µg/ml LDL-protein + 10 µM Cu⁺⁺ wurden bei 37° C in 2 ml TN-Puffer (20 mM Tris ph 8, 150 mM NaCl) in An- und Abwesenheit von 10 µM Testsubstanz inkubiert. Die Bildung von LDL-Oxidationsprodukten wurde anhand thiobarbitursäurereaktiver Substanzen (TBARS) bei OD532 16 und 23 Stunden nach Beginn der Inkubation gemessen.

**Tabelle 2**

| Inhibition der LDL-Oxidation durch Lipoxydase | |
|---|---|
| Testsubstanz | IC50 (µM) |
| Probucol | 10 |
| Verb. gem. Bsp. 1 | 0.05 |

100 µg LDL-Protein und 500 U/ml Lipoxydase aus Sojabohne (Sigma, München) wurden bei 37° C in TN-Puffer (50 mM Tris pH 8, 150 mM NaCl in An- und Abwesenheit von 0-10 µM Testsubstanz inkubiert. Die Bildung von Dienen aus der Oxidation ungesättigter Fettsäuren des LDL wird bei A 234 über 3 h verfolgt und ist im Kontrollansatz ca. 0.075/h. Die Inhibition der Dienbildung dient als Maß der Lipoxydaseaktivität am LDL-substrat.

**Tabelle 3**

| Inhibition der LDL-Oxidation-abhängigen Cholesterinesterbildung im P388-Makrophagen | |
|---|---|
| Versuch | CE/FC (µg/µg) |
| Kontrolle ohne LDL | 0.44 |
| Kontrolle + LDL | 1.81 |
| Kontrolle + LDL + 10 µm Probucol | 0.52 |
| Kontrolle + LDL + 10 µm Bsp. 1 | 0.48 |

125 µg/ml LDL-Protein wurde in F 10-Medium +1 µM Cu⁺⁺ für 5 Stunden inkubiert, um LDL-Antioxidantien zu depletieren. Dann wurden P388D.1 murine Makrophagen (1 x 10⁶ Zellen/ml) in F 10-Medium + 10 % FCS für 18 Stunden mit diesem Ansatz weiter inkubiert. Unter diesen Versuchsbedingungen bleibt das Verhältnis freies Cholesterin (FC)/Protein konstant bei 0.2 µg/mg, während die Cholesterinester in Abhängigkeit von LDL im Medium in den P388D.1 Makrophagen ansteigen, hier ausgedrückt als Anstieg des Verhältnisses von Cholesterinester/freiem Cholesterin (ED/FC).

**Tabelle 4**

| Reduktion des LDL-Katabolismus im arteriosklerotischen Arterienwandgewebe | | | | |
|---|---|---|---|---|
| Gruppe | Anzahl der Tiere n | Ausgewertete Läsionen n | Einbau von 131_{J}-TDS-LDLnorm±SEM ng/(mm³xdt/2) | U-Test |
| Kontrolle | 6 | 43 | 65.7 ± 3.9 (20-131 | |
| Verb. gem. Bsp.1 | 6 | 48 | 35 ± 1,9 (7-63) | p<0.001 |
| Probucol | 3 | 20 | 84 ± 7.5 (40-144) | n.s. |

Jeweils 6 Kaninchen in der Kontrollgruppe und Bsp.l-Gruppe, bzw. 3 Kaninchen in der Probucol-Gruppe für 3 Monate mit 0.5 % Cholesterin-fettreicher Kaninchendiät gefüttert. Die Tiere der verschiedenen Gruppen haben S-Cholspiegel von 1028±99 (771-1358) - 1155±200 (655-1787) mg % am Ende der Fütterungsphase und- ein Körpergewicht von 3050-3680 g erreicht. Die Tiere erhalten dann für 10 Tage 0.2 % Testsubstanz in der fettreichen Diät und anschließend eine Infusion von ¹³¹J-Deoxysorbitol-LDL. Der Einbau von ¹³¹J-Deoxysorbitol in den Arterienwandzellen erfolgt ganz überwiegend in der arteriosklerotischen Läsion und ermöglicht die Quantifizierung des LDL-Umsatzes in den Zellen. 24 Stunden nach der Infusion des LDL werden die Tiere getötet und die thorakale Aorta präpariert. Es werden Transversalschnitte angefertigt, die im Gewebe gespeicherte Radiojodaktivität gemessen, eine Autoradiographie durchgeführt und die Plaquevolumina jedes Transversalschnittes morphometrisch gemessen. Der Einbau von LDL ist in ng/mm³ x d angegeben.

## Patentansprüche

1. Arzneimittel, enthaltend neben üblichen Träger- und Hilfsstoffen mindestens eine Verbindung der Formel I 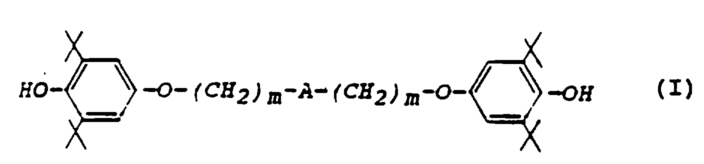 in der
m eine ganze Zahl zwischen 1 und 6 und
A eine Bindung, eine Methylengruppe, eine Ketogruppe oder eine Gruppe -CH(OH)-
bedeuten,
mit Ausnahme der Verbindung 1,2-Bis-[4-hydroxy-3,5-di-tert.butyl-phenoxy]ethan

2. Verwendung von Verbindungen der Formel I gemäß Anspruch 1 zur Herstellung von pharmazeutischen Zusammensetzungen mit antiatherosclerotischen Wirkung.

3. Verbindungen der Formel I 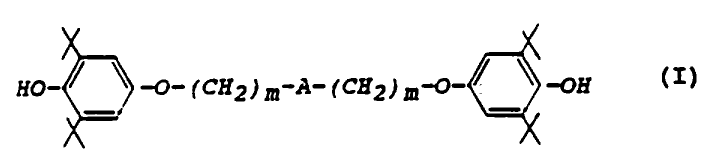 in der
m eine ganze Zahl zwischen 1 und 6 und
A eine Bindung, eine Methylengruppe, eine Ketogruppe oder eine Gruppe -CH(OH)-
bedeuten,
wobei für den Fall, daß A eine Bindung darstellt, m nicht die Zahl 1, 2 oder 3 bedeuten darf.

4. Verfahren zur Herstellung von Verbindungen der Formel I 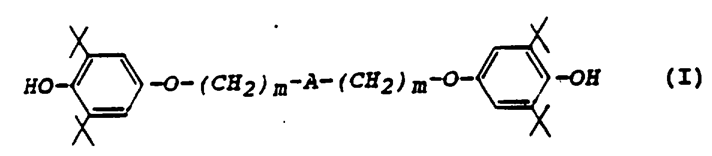 in der
m eine ganze Zahl zwischen 1 und 6 und
A eine Bindung, eine Methylengruppe, eine Ketogruppe oder eine Gruppe -CH(OH)-
bedeuten,
wobei für den Fall, daß A eine Bindung darstellt, m nicht die Zahl 1, 2 oder 3 bedeuten darf,
dadurch gekennzeichnet, daß man in an sich bekannter Weise entweder
a) zwei Mol 3,5-Di-tert.butyl-hydrochinon mit einem Mol einer Verbindung der Formel (III)
X-(CH₂)ₘ-A-(CH₂)ₘ-X (III)
in der X eine reaktive gruppe darstellt, A und m die oben angegebene Bedeutung haben,
umsetzt, oder
b) zunächst ein Mol 3,5-Di-tert.butyl-hydrochinon mit einem Mol einer Verbindung der Formel IV
X-(CH₂)ₘ-A-(CH₂)ₘ-Y (IV)
in der X, A und m die oben angegebene Bedeutung haben, und Y einen in die Gruppe X umwandelbaren Rest darstellt, umsetzt, und anschließend die erhaltene Verbindung nach Umwandlung der Gruppe Y in die Gruppe X mit einem weiteren Mol 3,5-Di-tert.butyl-hydrochinon umsetzt,
oder
c) für den Fall, daß m=1 und A die Gruppe CHOH bedeuten, 3,5-Di-tert.butyl-hydrochinon mit Epichlorhydrin oder Epibromhydrin zur Verbindung der Formel VI 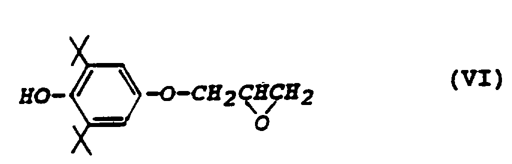 und diese Verbindung mit einem weiteren Mol 3,5-Di-tert.butyl-hydrochinon zur gewünschten Verbindung der Formel I umsetzt,

## Claims

1. Medicaments containing, besides usual carrier and adjuvant materials, at least one compound of the formula I 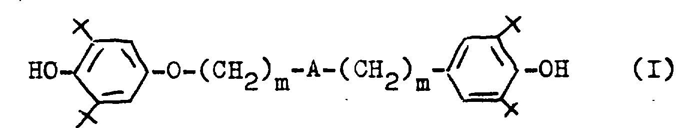 in which m signifies a whole number between 1 and 6 and A a bond, a methylene group, a keto group or a group -CH(OH)-, with the exception of the compound 1,2-bis-[4-hydroxy-3,5-di-tert.-butylphenoxy]-ethane.

2. Use of compounds of the formula I according to claim 1 for the preparation of pharmaceutical compositions with anti-atherosclerotic action.

3. Compounds of the formula I 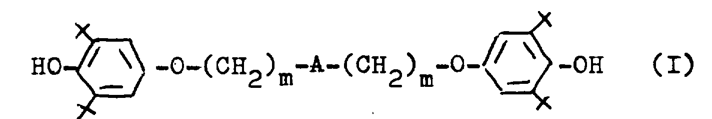 in which m signifies a whole number between 1 and 6 and A a bond, a methylene group, a keto group or a group -CH(OH)-, whereby, for the case that A represents a bond, m may not be the number 1, 2 or 3.

4. Process for the preparation of compounds of the formula I 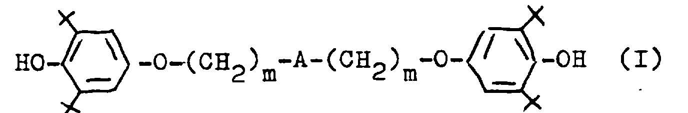 in which m signifies a whole number between 1 and 6 and A a bond, a methylene group, a keto group or a group -CH(OH)-, whereby, for the case that A represents a bond, m may not be the number 1, 2 or 3, characterised in that, in per se known manner, one either
a) reacts two moles of 3,5-di-tert.-butylhydroquinone with one mole of a compound of the formula (III)
X-(CH₂)ₘ-A-(CH₂)ₘ-X (III)
in which X represents a reactive group, A and m have the above-given meaning, or
b) first reacts one mole of 3,5-di-tert.-butylhydroquinone with one mole of a compound of the formula IV
X-(CH₂)ₘ-A-(CH₂)ₘ-Y (IV)
in which X, A and m have the above-given meaning and Y represents a residue convertible into the group X, and subsequently, after conversion of the group Y into the group X, reacts the compound obtained with a further mole of 3,5-di-tert.-butylhydroquinone, or c) for the case that m = 1 and A signifies the group CHOH, reacts 3,5-di-tert.-butylhydroquinone with epichlorohydrin or epibromohydrin to give the compound of the formula VI 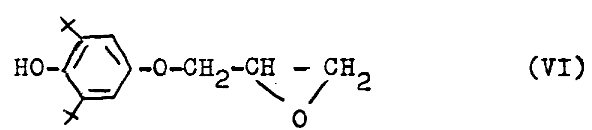 and reacts this compound with a further mole of 3,5-di-tert.-butylhydroquinone to give the desired compound of the formula I and subsequently, if desired, reduces a compound with A = CO or CH(OH) or, on the other hand, oxidises a compound with A = CH₂ or CH(OH).

## Revendications

1. Médicament contenant, en plus de véhicules et adjuvants usuels, au moins un composé de formule I 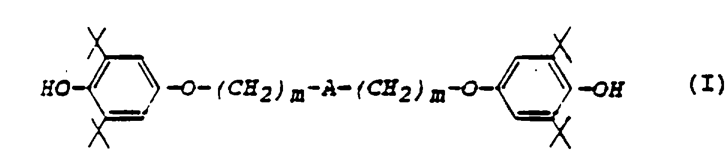 dans laquelle
m représente un nombre entier compris entre 1 et 6, et
A représente une valence libre, un groupe méthylène, un groupe céto ou un groupe -CH(OH)-
à l'exception du composé 1,2-bis-[4-hydroxy-3,5-di-tert.-butyl-phénoxy]-éthane.

2. Utilisation de composés de formule I selon la revendication 1, pour la préparation de compositions pharmaceutiques ayant une activité antiathéroscléreuse.

3. Composés de formule I 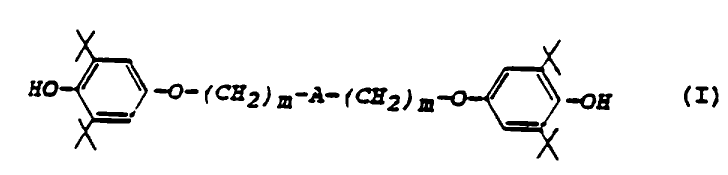 dans laquelle
m représente un nombre entier compris entre 1 et 6, et
A représente une valence libre, un groupe méthylène, un groupe céto ou un groupe -CH(OH)-,
étant entendu que, dans le cas où A représente une valence libre, m ne doit pas représenter le nombre 1, 2 ou 3.

4. Procédé de préparation de composés de formule I 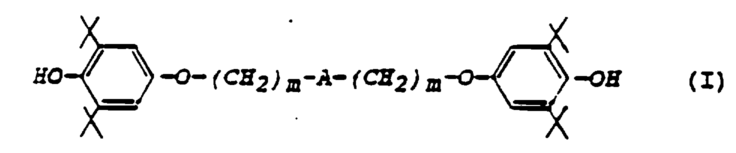 dans laquelle
m représente un nombre entier compris entre 1 et 6, et
A représente une valence libre, un groupe méthylène, un groupe céto ou un groupe -CH(OH)-,
étant entendu que dans le cas où A représente une valence libre, m ne doit pas représenter le nombre 1, 2 ou 3,
caractérisé par le fait que, de manière connue en soi,
a) on fait réagir deux moles de 3,5-di-tert.-butyl-hydroquinone avec une mole d'un composé de formule (III)
X-(CH₂)ₘ-A-(CH₂)ₘ-X (III)
dans laquelle X représente un groupe réactif, A et m ont les significations mentionnées ci-dessus, ou
b) on fait réagir d'abord une mole de 3,5-di-tert.-butyl-hydroquinone avec une mole d'un composé de fromule IV
X-(CH₂)ₘ-A-(CH₂)ₘ-Y (IV)
dans laquelle X, A et m ont les significations mentionnées ci-dessus et Y représente un reste qui peut être transformé en le groupe X, et ensuite, après transformation du groupe Y en le groupe X, on fait réagir le composé obtenu avec une mole supplémentaire de 3,5-di-tert.-butyl-hydroquinone, ou
c) dans le cas où m=1 et A représente le groupe CHOH, on fait réagir la 3,5-di-tert.-butyl-hydroquinone avec l'épichlorhydrine ou l'épibromhydrine pour obtenir le composé de formule VI 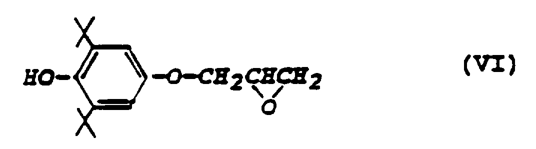 et on fait réagir ce composé avec une mole supplémentaire de 3,5-di-tert.-butyl-hydroquinone pour obtenir le composé de formule recherché, et ensuite, on réduit éventuellement un composé où A=CO ou CH(OH), ou inversement, on oxyde un composé où A=CH₂ ou CH(OH).
